Europäisches Patentamt

European Patent Office

Office européen des brevets

(11) Veröffentlichungsnummer: **0 170 010**
**B1**

(12) # EUROPÄISCHE PATENTSCHRIFT

(45) Veröffentlichungstag der Patentschrift:
**19.11.87**

(51) Int. Cl.⁴: **A 61 M 37/00,** A 61 F 13/02

(21) Anmeldenummer: **85107088.8**

(22) Anmeldetag: **08.06.85**

(54) **Selbstklebendes Pflaster.**

(30) Priorität: **23.06.84 DE 3423328**

(43) Veröffentlichungstag der Anmeldung:
**05.02.86 Patentblatt 86/6**

(45) Bekanntmachung des Hinweises auf die Patenterteilung:
**19.11.87 Patentblatt 87/47**

(84) Benannte Vertragsstaaten:
**AT BE CH DE FR GB IT LI NL SE**

(56) Entgegenhaltungen:
**CH - A - 338 554**
**CH - A - 341 948**
**DE - A - 3 202 775**

(73) Patentinhaber: **Beiersdorf Aktiengesellschaft,
Unnastrasse 48, D-2000 Hamburg 20 (DE)**

(72) Erfinder: **Nick, Erich, Dr., Eichhörnchenweg 2,
D-2080 Pinneberg (DE)**
Erfinder: **Guse, Günter, Dr. Dipl.-Chem.,
Liliencronstrasse 30, D-2000 Hamburg 73 (DE)**
Erfinder: **Asmussen, Bodo, Dr., Dorotheenweg 1a,
D-2071 Ammerbek (DE)**

## Beschreibung

Die Erfindung betrifft ein selbstklebendes Pflaster mit separaten Wirkstoff-Segmenten auf einem Träger zur transdermalen Applikation.

Pflaster für die transdermale Applikation von Wirkstoffen sind bekannt. Neben einigen im Handel befindlichen derartigen Pflastern gibt es eine Vielzahl von Publikationen und Patenten dazu. Darin werden grundsätzlich verschiedene Wege beschritten.

Einer dieser Wege besteht darin, den Wirkstoff in einem Reservoir anzuordnen, das mit einer Steuermembran gegenüber der Haut abgeschirmt ist und durch diese eine kontrollierte Menge Wirkstoff in die Haut abgibt. Wirkstoff, Reservoir-Bestandteile und Membran müssen sorgfältig aufeinander abgestimmt sein, so dass dieses System nicht für beliebige Wirkstoffe einsetzbar ist. Zudem ist die Herstellung eines solchen Pflasters aufwendig, bedingt durch den komplizierten Aufbau.

Einen grundsätzlich anderen Weg beschreiten Systeme, bei denen der Wirkstoff in die Klebemasse des Pflasters eingearbeitet ist. Auch hier ist es erforderlich, Wirkstoff und Klebemasse sorgfältig aufeinander abzustimmen. Obwohl diese Systeme einfacher und wirtschaftlicher herzustellen sind, bedingen sie zwangsläufig Interaktionen zwischen Wirkstoff und Kleber, zumal in aller Regel Klebstoffe nicht aus definierten, einzelnen Bestandteilen bestehen, sondern vielfältige Mischungen darstellen.

Ferner beschreibt die DE-OS 3 202 775 ein Adhäsions-Pflaster zur transdermalen Applikation eines Wirkstoffs, bei dem der Wirkstoff in Form von separaten angenähert kalottenförmigen Segmenten auf die Klebefläche des Pflasters aufgedruckt ist. Obwohl ein solches Pflaster wirtschaftlich herstellbar ist, hat es doch gewichtige Nachteile und konnte sich demgemäss nicht in der Praxis durchsetzen. Insbesondere die Interaktionen zwischen Wirkstoff und Klebemasse beeinträchtigen eine gleichmässige Langzeitabgabe vom Wirkstoff durch die Haut aufgrund gegenseitiger Durchwanderungen, insbesondere von Wirkstoff in die Klebemasse, von Weichmachern oder weichmacherähnlichen Substanzen in die Wirkstoff-Segmente je nach äusseren Einflüssen, wie Temperatur und Feuchtigkeit. Den strengen Vorschriften zur behördlichen Zulasssung als Arzneimittel kann ein solches Pflaster nicht genügen, insbesondere auch im Hinblick auf verlangte Lagerstabilität unter möglicherweise erschwerten Bedingungen, bei der Migrationserscheinungen über längere Zeiträume ablaufen können.

Aufgabe der Erfindung war es demgemäss, ein selbstklebendes Pflaster zur transdermalen Applikation zu schaffen, das die Nachteile des Standes der Technik nicht oder in wesentlich geringerem Umfang aufweist. Insbesondere war ein Pflaster zu schaffen, das einerseits wirtschaftlich herstellbar ist, das andererseits aber frei von Problemen der Migration von Klebstoff in Wirkstoff und umgekehrt ist, zudem möglichst jede Art von Interaktion der beiden Komponenten vermeidet.

Demgemäss betrifft die Erfindung ein selbstklebendes Pflaster mit separaten angenähert kalottenförmigen Wirkstoff-Segmenten auf einem Träger zur transdermalen Applikation, das dadurch gekennzeichnet ist, das auf dem Träger räumlich von den Wirkstoff-Segmenten separate Klebstoff-Segmente angeordnet sind, die aus solchen Klebstoff-Systemen bestehen, die sich als Dispersion, Plastisol oder Organosol verarbeiten lassen, und dass sowohl die Klebstoff-Segmente als auch die Wirkstoff-Segmente mit ihrer Basis auf dem Träger haften.

Besonders vorteilhaft ist es, dass die Scheitelpunkte der Wirkstoff- und Klebstoff-Segmente gegenüber dem Träger etwa gleiche Höhe haben. Damit wird ein gleichmässig guter Kontakt zur Hautoberfläche beider Elemente erreicht.

Damit ist das erfindungsgemässe Pflaster frei von Interaktion zwischen Wirkstoff und Kleber. Der daraus resultierende Vorteil besteht insbesondere darin, dass als Kleber an sich bekannte Kleber vom Dispersionstyp und dergleichen verwendet werden können, dass andererseits der Wirkstoff mit geeigneten pharmazeutischen Hilfsstoffen verarbeitet werden kann, die dem Fixieren auf dem erfindungsgemässen Pflaster und seiner optimalen Diffusion in die Haut dienlich sind. Es ist damit nicht mehr notwendig, eine auf den jeweiligen Wirkstoff zugeschnittene Klebstoffgalenik und umgekehrt eine auf den Klebstoff zugeschnittene Wirkstoff-Galenik zu entwickeln. Vielmehr resultiert ein ausserordentlich variables System, mit dem Wirkstoffe der verschiedensten Art der transdermalen Applikation zugänglich werden.

Vorteilhaft sind sowohl die Klebstoff-Segmente als auch die Wirkstoff-Segmente jeweils aus Dispersionen mit hohem Feststoffgehalt im Druckverfahren aufgetragen. Als Druckverfahren eignen sich dabei der Tiefdruck, insbesondere aber der Siebdruck, der sich durch einfache Handhabung und ausserordentliche Wirtschaftlichkeit bei exakter Anordnung der Segmente auszeichnet.

Bevorzugt sind die Klebstoff-Segmente und die Wirkstoff-Segmente in regelmässiger Folge angeordnet, bedingt durch die Geometrie der Siebdruck-Vorrichtungen. Dabei haben die Kalotten vorzugsweise einen Basisdurchmesser von bis zu etwa 5000 µm, insbesondere 200-1500 µm. Falls erwünscht, lassen sich jedoch durchaus auch grössere Kalotten herstellen.

Aufgrund der räumlichen Trennung von Wirkstoff und Kleber kann der Kleber aus an sich bekannten hautfreundlichen Klebern bestehen, die wie oben angegeben, verarbeitbar sind. Geeignet sind unter anderem Kautschuk, Polyacrylsäureester oder Polyisobutylen, ggf. zusammen mit klebrigmachenden Harzen. Diese Kleber werden vorteilhaft aus wässerigen, konzentrierten, thixotropen Haftklebe-Dispersionen verarbeitet, wobei der Feststoffgehalt vorzugsweise bei 55-65 Gew.-% liegt. Beispielsweise geeignet sind Haftkleber auf der Basis von (Meth)-Acrylsäureestern mit Alkylresten von 4-18 C-Atomen, wie Acrylsäure-Butylester, Acrylsäure-Äthylhexylester oder Acrylsäure-Stearylester, vernetzt oder unvernetzt, wobei eine Vernetzung ggf. durch Elektronenstrahlen bewirkt sein kann.

Das Prinzip des Rotationssiebdrucks besteht in der Verwendung einer rotierenden, nahtlosen, trommelförmigen und perforierten Rundschablone. Im Innenmantel presst eine mechanisch oder magnetisch gehaltene Rund- oder Vierkantrakel die in die Trommel eingespeiste Dispersion durch die Perforation der Schablonenwand auf die Trägerbahn. Diese wird mit einer Geschwindigkeit geführt, die der Umfangsgeschwindigkeit der rotierenden Siebtrommel entspricht, mittels einer Gegendruckwalze gegen den Aussenmantel der Siebtrommel. Zur Herstellung der erfindungsgemässen Pflaster werden dabei in geeigneter Weise zwei hintereinander geschaltete, synchron laufende Siebdruckwerke verwendet, die Klebstoff und dann Wirkstoff entsprechend der Lochgeometrie der Siebe passgenau nebeneinander plazieren. Grösse und Dosierung der sich als Kalotten ausbildenden mikrokleinen Kleb- und Wirkstoffflächen lässt sich durch den Durchmesser des Sieblochs und der Wandstärke des Siebes genau bestimmen. Die Zahl der Möglichkeiten der Anordnung ist unbegrenzt und kann je nach Wunsch gewählt werden. Damit ergibt sich zudem als wirtschaftlicher Vorteil der Herstellungsweise, dass Klebstoff- und Wirkstoffzubereitung in einer Linie aufgebracht werden können, und dies mit hoher Dosierungsgenauigkeit.

Die Dispersion sollte gelartig bis pastös sein und nach Auftrocknung einen elastischen Film bilden, z.B. durch Zusatz von Polyvinylalkohol, Polyvinylpyrrolidon, wasserlöslichen Cellulosederivaten oder anderen mehr oder weniger wasserlöslichen Verfilmungsmitteln. Auch mikroverkapselte Stoffe können über die Dispersion verarbeitet werden.

Weiterhin ist es möglich, membranbildende Stoffe separat durch Zuschaltung eines weiteren Siebdruckwerkes in Linie passergenau auf die Wirksubstanz aufzubringen, ohne den Klebeeffekt zu stören.

Soll eine Elektronenstrahl-Vernetzung erfolgen, so geschieht dies vorteilhaft nach dem Klebstoffauftrag und vor dem Wirkstoffauftrag, ohne die Produktionslinie zu unterbrechen.

Abschliessend wird die Beschichtung vorteilhaft in einem Heissluftkanal oder durch Infrarotbzw. Hochfrequenzstrahlung getrocknet. Als Träger kommen insbesondere diffusionsdichte Folien in Betracht. Geeignet sind insbesondere Träger, auf denen Klebstoff und Wirkstoffzubereitung ohne Hilfsmittel haften. Gegebenenfalls kann die Oberfläche des Trägers aber auch zusätzlich behandelt werden, z.B. durch Korona-Entladung oder Beschichtung mit einem Haftvermittler, der als Primer die Verankerung vermittelt.

Zur transdermalen Applikation geeignete Wirkstoffe sind vielfältig bekannt. So beschreibt die EP-OS 72 251 eine Vielzahl solcher Wirkstoffe. Als Beispiele seien die folgenden genannt:

| | |
|---|---|
| Antihypertonika | Calciumantagonisten |
| Antihypotonika | Antiemetika |
| Vasodilatantien | Antitussiva |
| β-Blocker | Sedativa |
| Analgetika | Antihystaminika |
| Psychopharmaka | Hormone |
| Antiasthmatika | Antibiotika |
| Antirheumatika | Cytostatika |
| Antiarythmika | |

In den Figuren wird die Erfindung beispielhaft erläutert, ohne dass damit eine Beschränkung gemeint ist. Vielmehr kann der Fachmann geeignete Änderungen aufgrund seines Fachwissens vornehmen, ohne damit den Rahmen der Erfindung zu verlassen.

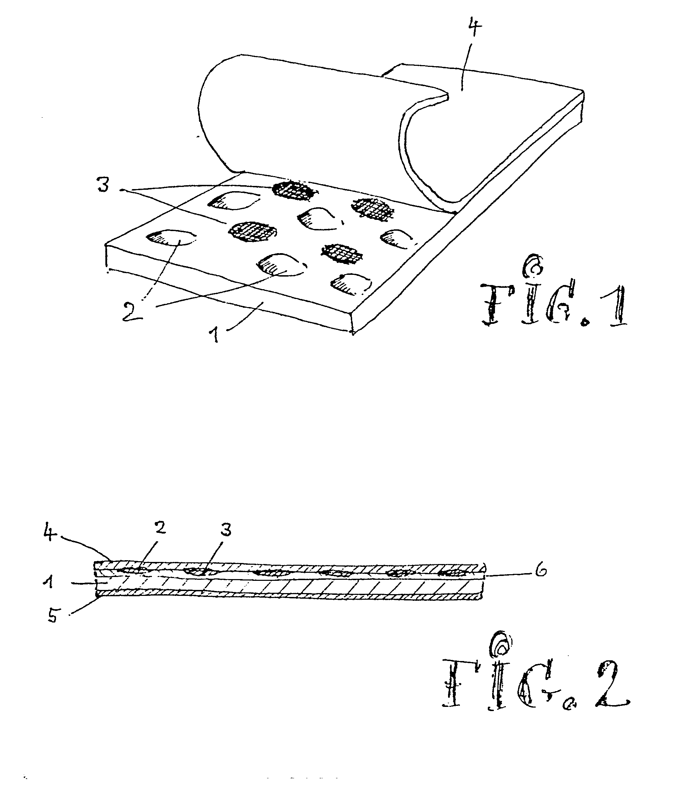

Fig. 1 zeigt ein erfindungsgemässes selbstklebendes Pflaster, Fig. 2 einen Querschnitt durch ein solches. Auf einem Träger 1 befinden sich gem. Fig. 1 kalottenförmige Klebstoff-Segmente 2 und Wirkstoff-Segmente 3, die mittels einer Abdeckung 4 teilweise abgedeckt sind, teilweise ist diese Abdeckung 4 angehoben. Vorteilhaft ist die Abdeckung 4 an der Seite klebstoffabweisend ausgerüstet, die den Klebstoff-Segmenten 2 und Wirkstoff-Segmenten 3 zugewandt ist, sofern die Abdeckung 4 nicht selbst bereits klebstoffabweisend aus sich heraus ist. Fig. 2 zeigt demgegenüber eine ergänzte Variante mit Träger 1, Klebmasse-Segmenten 2 und Wirkstoff-Segmenten 3, eingebettet in einen Haftvermittler 6 und abgedeckt mit einem Trennfilm 4. An der Unterseite des Trägers 1 befindet sich ein Decklack 5, der aus einer Lackschicht oder einer aufgedampften Metallschicht bestehen kann.

*Beispiel 1:*

*A. Herstellung einer Wirkstoff-Zubereitung*

27,35 Gewichtsteile Hydroxypropylmethylcellulose (Pharmacoat 603) werden in ein Gemisch von 30 GT Isopropanol, 30 GT Wasser und 8,20 GT 1,2-Propylenglycol kalt eingerührt. Das Gemisch wird unter ständigem Rühren auf 50° C erwärmt, bis eine klare Lösung entstanden ist. In diese werden 4,45 GT micronisiertes Moxonidin sorgfältig eingemischt.

*B. Herstellung einer Klebstoffzubereitung*

100 GT einer handelsüblichen wässerigen Polymerdispersion auf Acrylsäureester-Basis (Acronal 80 D) werden durch Zusatz von 4 GT der 16%igen ammoniakalisch-wässerigen Lösung einer Polyarylsäure (Collacral P) auf eine Viscosität von etwa 200 Pas verdickt.

*C. Druckvorgang*

Eine einseitig aluminisierte und hautfarben lakkierte Polyethylenterephthalat-Folie von 15 μm Stärke (Hostaphan RN 15) wird durch ein erstes rotierendes Siebdruckwerk bei einer Bahngeschwindigkeit von 20 m/Min mit der Wirkstoffzubereitung beschichtet. Dabei wird ein Siebzylinder verwendet, der Punktreihen in Bahnlängsrichtung erzeugt. Jeder Punkt hat einen Basisdurchmesser von etwa 400 μm; der Abstand zwischen den Punkten beträgt in Bahnlängsrichtung etwa 100 μm, in Querrichtung etwa 600 μm. Die Auftragsmenge wird über die Rakeleinstellung so geregelt, dass kalottenförmige Punkte entstehen, die im getrockneten Zustand eine Höhe von etwa 160 µm besitzen. Das entspricht einer Auftragsmenge von etwa 25 g Wirkstoffzubereitung je m² Träger. Die Trocknung erfolgt in einem Warmluftkanal von 6 m Länge bei etwa 70° C. Ein zweites rotierendes Siebdruckwerk gleichen Typs druckt anschliessend Punktreihen der Klebstoffzubereitung zwischen die getrockneten wirkstoffhaltigen Punktreihen. Dabei werden Punktgrösse, Punkthöhe und somit auch die Gesamtauftragsmenge auf die gleichen Werte wie bei der Wirkstoffzubereitung eingeregelt. Hinter diesem zweiten Druckwerk folgt erneut eine Trockenstrecke des beschriebenen Typs und anschliessend ein Kaschierwerk, in dem die Bahn mit einem wiederentfernbaren Schutzfilm abgedeckt wird, beispielsweise einer einseitig aluminisierten und siliconisierten Polyethylenterephthalat-Folie von 100 µm Stärke (Hostaphan RN 100).

### D. Konfektionierung

Die zweifach bedruckte, getrocknete und abgedeckte Bahn wird nun in Einzelpflaster beliebiger Grösse aufgeteilt, je nach der erwünschten Wirkstoffdosierung. Wählt man beispielsweise eine Grösse von 25 cm³, so hat man je etwa 5000 Wirkstoff- und Klebstoffpunkte, entsprechend einem Gehalt von 7 mg Moxonidin. Diese Pflaster werden einzeln in ein diffusionsdichtes Primärpackmittel eingeschweisst, beispielsweise einen Flachbeutel aus PE/Alu/Papier-Verbundmaterial.

### Patentansprüche

1. Selbstklebendes Pflaster mit separaten angenähert kalottenförmigen Wirkstoff-Segmenten auf einem Träger zur transdermalen Applikation, dadurch gekennzeichnet, dass auf dem Träger (1) räumlich von den Wirkstoff-Segmenten (3) separate angenähert kalottenförmige Klebstoff-Segmente (2) angeordnet sind, die aus solchen Klebstoffsystemen bestehen, die sich als Dispersion, Plastisol oder Organosol verarbeiten lassen, und dass sowohl die Klebstoff-Segmente (2) als auch die Wirkstoff-Segmente (3) mit ihrer Basis auf dem Träger (1) haften.

2. Pflaster nach Anspruch 1, dadurch gekennzeichnet, dass die Scheitelpunkte der Wirkstoff-Segmente (3) und der Klebstoff-Segmente (2) etwa gleiche Höhe gegenüber dem Träger (1) haben.

3. Pflaster nach Anspruch 1 oder 2, dadurch gekennzeichnet, dass sowohl die Klebstoff-Segmente (2) als auch die Wirkstoff-Segmente (3) jeweils aus Dispersionen mit hohem Feststoffgehalt im Druckverfahren vom Typ Tiefdruck oder insbesondere Siebdruck aufgetragen sind.

4. Pflaster nach einem der Ansprüche 1-3, dadurch gekennzeichnet, dass die Klebstoff-Segmente (2) und die Wirkstoff-Segmente (3) in regelmässiger Folge angeordnet sind und einen Kalotten-Basisdurchmesser von bis zu etwa 5000 µm haben, insbesondere 200-1500 µm.

5. Pflaster nach einem der Ansprüche 1-4, dadurch gekennzeichnet, dass die Klebstoff-Segmente (2) aus einem hautfreundlichen Kleber z.B. auf Basis Kautschuk, Polyacrylsäureester oder Polyisobutylen bestehen, ggf. zusammen mit klebrigmachenden Harzen.

6. Pflaster nach einem der Ansprüche 1-5, dadurch gekennzeichnet, dass die Klebstoff-Segmente (2) aufgetragen sind aus wässerigen, thixotropen Haftklebe-Dispersionen mit einem Feststoffgehalt von 55-65 Gew.-%.

7. Pflaster nach einem der Ansprüche 1-6, dadurch gekennzeichnet, dass die Klebstoff-Segmente (2) nachträglich vernetzt sind, z.B. durch Elektronenstrahlen.

8. Pflaster nach einem der Ansprüche 1-7, dadurch gekennzeichnet, dass die Wirkstoff-Segmente (3) mindestens einen Wirkstoff, ggf. neben geeigneten pharmazeutischen Hilfs- und Trägerstoffen in für die transdermale Applikation geeigneter Form enthalten.

9. Pflaster nach einem der Ansprüche 1-8, dadurch gekennzeichnet, dass der Träger (1) für den Wirkstoff undurchlässig ist und ggf. mit einem Haftvermittler für die Segmente (2) und (3) und/oder einem aussenliegenden Decklack (5) oder dergleichen beschichtet ist.

10. Pflaster nach einem der Ansprüche 1-9, dadurch gekennzeichnet, dass der Wirkstoff 2-Methyl-4-chlor-6-methoxy-5-(2-imidazolin-2-yl)-amino-pyrimidin[Moxonidin] ist.

11. Pflaster nach einem der Ansprüche 1-10, dadurch gekennzeichnet, dass ein Teil der Wirkstoff-Segmente (3) einen Wirkstoff, der Rest einen anderen Wirkstoff enthält.

### Claims

1. Self-adhesive plaster with separate, approximately spherical cap-shaped active compound segments on a carrier for transdermal administration, characterized in that, on the carrier (1) and spatially separated from the active compound segments (3), approximately spherical cap-shaped adhesive segments (2) are arranged, which consist of types of adhesive systems which can be processed as a dispersion, plastisol or organosol, and that both the adhesive segments (2) and the active compound segments (3) adhere with their base to the carrier (1).

2. Plaster according to Claim 1, characterized in that the apices of the active compound segments (3) and of the adhesive segments (2) have approximately the same height relative to the carrier (1).

3. Plaster according to Claim 1 or 2, characterized in that both the adhesive segments (2) and the active compound segments (3) have each been applied from high-solids dispersions by a printing process of the gravure printing or especially screen printing type.

4. Plaster according to one of Claims 1-3, characterized in that the adhesive segments (2) and the active compound segments (3) are arranged in a regular sequence and have a base diameter of the spherical caps of up to about 5000 µm, especially 200-1500 µm.

5. Plaster according to one of Claims 1-4, characterized in that the adhesive segments (2) consist of a skin-tolerated adhesive, for example based on rubber, polyacrylate ester or polyisobutylene, if appropriate together with tackifying resins.

6. Plaster according to one of Claims 1-5, characterized in that the adhesive segments (2) have been applied from aqueous, thixotropic pressure-sensitive adhesive dispersions of 55-65% by weight solids content.

7. Plaster according to one of Claims 1-6, characterized in that the adhesive segments (2) have been postcrosslinked, for example by means of electron beams.

8. Plaster according to one of Claims 1-7, characterized in that the active compound segments (3) contain at least one active compound, if appropriate in addition to suitable pharmaceutical auxiliaries and excipients in a form suitable for transdermal administration.

9. Plaster according to one of Claims 1-8, characterized in that the carrier (1) is impermeable for the active compound and, if appropriate, is coated with an adhesion promoter for the segments (2) and (3) and/or an external top coat (5) or the like.

10. Plaster according to one of Claims 1-9, characterized in that the active compound is 2-methyl-4-chloro-6-methoxy-5-(2-imidazolin-2-yl)-amino-pyrimidine[moxonidine].

11. Plaster according to one of Claims 1-10, characterized in that one part of the active compound segments (3) contains one active compound and the remainder contains another active compound.

## Revendications

1. Emplâtre auto-adhésif comprenant des segments de principe actif distincts dont la forme s'approche de celle d'une calotte, sur un support pour l'administration transdermique, caractérisé en ce que des segments d'adhésif (2) distincts dont la forme s'approche de celle d'une calotte, qui consistent en systèmes d'adhésif qui peuvent être mis en œuvre à l'état de dispersion, de plastisol ou d'organosol, sont disposés à l'écart de segments de principe actif (3) sur le support (1) et que tant les segments d'adhésif (2) que les segments de principe actif (3) adhèrent au support (1) par leur base.

2. Emplâtre suivant la revendication 1, caractérisé en ce que les sommets des segments de principe actif (3) et des segments d'adhésif (2) ont à peu près la même hauteur par rapport au support (1).

3. Emplâtre suivant la revendication 1 ou 2, caractérisé en ce que tant les segments d'adhésif (2) que les segments de principe actif (3) sont appliqués à partir de dispersions à haute teneur en solides suivant un procédé d'impression du type en creux ou, en particulier, sérigraphique.

4. Emplâtre suivant l'une quelconque des revendications 1 à 3, caractérisé en ce que les segments d'adhésif (2) et les segments de principe actif (3) sont agencés en succession régulière et ont un diamètre à la base de la calotte jusqu'à environ 5000 µm, en particulier de 200-1500 µm.

5. Emplâtre suivant l'une quelconque des revendications 1 à 4, caractérisé en ce que les segments d'adhésif (2) consistent en un adhésif compatible avec la peau, par exemple à base de caoutchouc, d'ester d'acide polyacrylique ou de polyisobutylène, éventuellement avec des résines conférant de l'adhésivité.

6. Emplâtre suivant l'une quelconque des revendications 1 à 5, caractérisé en ce que les segments d'adhésif (2) ont été appliqués à partir de dispersions aqueuses thixotropes de colle de contact ayant une teneur en solides de 55-65% en poids.

7. Emplâtre suivant l'une quelconque des revendications 1 à 6, caractérisé en ce que les segments d'adhésif (2) ont été ultérieurement réticulés, par exemple au moyen de faisceaux d'électrons.

8. Emplâtre suivant l'une quelconque des revendications 1 à 7, caractérisé en ce que les segments de principe actif (3) contiennent au moins un principe actif, éventuellement avec des adjuvants ou excipients pharmaceutiquement appropriés sous une forme propre à l'administration transdermique.

9. Emplâtre suivant l'une quelconque des revendications 1 à 8, caractérisé en ce que le support (1) est imperméable pour le principe actif et est éventuellement revêtu d'un intermédiaire d'adhérence pour les segments (2) et (3) et/ou d'une couche de couverture extérieure (5) ou analogue.

10. Emplâtre suivant l'une quelconque des revendications 1 à 9, caractérisé en ce que le principe actif est la 2-méthyl-4-chloro-6-méthoxy-5-(2-imidazolin-2-yl)-aminopyrimidine [Monoxidine].

11. Emplâtre suivant l'une quelconque des revendications 1 à 10, caractérisé en ce qu'une partie des segments de principe actif (3) contient un principe actif et le reste contient un autre principe actif.

FIG.1

FIG.2